# EUROPEAN PATENT SPECIFICATION

(11) **EP 3 423 996 B1**
(45) Date of publication and mention of the grant of the patent: **12.02.2025**
(21) Application number: 17711864.3
(22) Date of filing: 01.03.2017
(51) Int. Cl.: G06Q 10/00, A61L 9/015

(54) **CARGO TRANSPORT SYSTEM FOR PERISHABLE PRODUCTS**
FRACHTTRANSPORTSYSTEM FÜR VERDERBLICHE PRODUKTE
SYSTÈME DE TRANSPORT DE MARCHANDISES POUR DES PRODUITS PÉRISSABLES

(30) Priority: 01.03.2016 US 201662301934 P
(43) Date of publication of application: 09.01.2019
(73) Proprietor: Carrier Corporation, Palm Beach Gardens, FL 33418 (US)
(72) Inventor: YASAR, Murat, East Hartford, Connecticut 06108 (US); LESHUK, Jeffrey Allen, Davis , CA 95618 (US); BEASLEY, Marc, Beverly, Massachusetts 01915 (US); CYWILKO, Mark E., Syracuse, New York 13221 (US)
(74) Representative: Dehns
(86) International application number: PCT/US2017/020073
(87) International publication number: WO 2017/151698

(56) References cited:
- WO-A1-95/22729
- WO-A2-03/004521
- US-A1- 2008 159 910
- US-A1- 2014 180 953
- US-A1- 2015 300 887
- US-B1- 7 455 225
- "GRUNDLAGEN DER REGELUNGSTECHNIK", 1 January 1991, SPRINGER VERLAG, DE, Berlin Heidelberg, ISBN: 978-3-540-17112-6, article G SCHMIDT: "GRUNDLAGEN DER REGELUNGSTECHNIK PASSAGE", pages: 291 - 293, XP055676644
- SCHRÜFER ET AL: "ELEKTRISCHE MESSTECHNIK", ELEKTRISCHE MESSTECHNIK. MESSUNG ELEKTRISCHER UNDNICHTELEKTRISCHER GROESSEN, XX, XX, 1 January 1988 (1988-01-01), pages 405 - 419,268, XP002197938

## Description

### BACKGROUND

The present disclosure relates to a computer implemented method of operating a product condition system.

Traditional cargo transport systems may monitor and collect environment parameter data such as temperature, humidity and ethylene concentrations during refrigerated transportation. The collected parameter data may be used to infer a condition of the perishable product in a very general manner. Typically, conclusions drawn from such data is speculative and that the product may have suffered due to sub-optimal environment parameters. Improvements in the transportation of perishable products is desirable.

US 2014/180953 discloses a method of controlling conditions within a container for distribution of perishable goods in response to changes in the scheduled distribution plan in order to attain a desired ripeness at the final destination. US 7,455,225 discloses a system for remote monitoring and managing of goods during transportation. US 2015/300887 discloses a system for monitoring the temperature of a food product using a temperature probe inserted in to the product, the temperature reading is then used to control a cooling system for that product.

### SUMMARY

According to a first aspect, the invention provides a computer implemented method of operating a product condition system comprising: measuring first and second conditions of a perishable product by respective first and second detectors, wherein the first condition of the perishable product is a gas emitted by the perishable product and the first detector is a gas detector, and a first condition signal associated with the measured first condition of the perishable product is sent from the first detector and to a detector data module, and wherein the second condition of the perishable product is a different condition type to that of the first condition of the perishable product; measuring a first environment parameter of a containment where the perishable product is located, wherein the first environment parameter is measured by an environment detector; comparing the first and second conditions of the perishable product to respective first and second pre-programmed thresholds respectively; determining a single environment parameter target when at least one of the first and second pre-programmed thresholds are met from a pre-programmed data table as a function of both of the measured first and second measured conditions of the perishable product, wherein the single environment parameter target is established to reduce degradation of the perishable product, and wherein the determining comprises accessing the pre-programmed data table indicative of a perishable product type by an analysis module and from a product information database, wherein the pre-programmed data table includes a plurality of condition types, a plurality of thresholds associated with each condition type of the plurality of condition types; and sending a command signal derived from the single environmental parameter target to an environmental control assembly to meet the first environment parameter target.

Optionally, the analysis module and the reporting module are software-based.

Optionally, the detector data module and the product information database are contained within a computer readable and writeable storage medium.

Optionally, the analysis module is supported by a microprocessor.

Optionally, the method includes measuring the second condition of the perishable product by the second detector; and comparing the second condition to a second pre-programmed threshold.

Optionally, the method includes determining a second environment parameter target when the second pre-programmed threshold is met.

Optionally, the perishable product is one type of a plurality of types of perishable products, and the data table is one of a plurality of data tables stored in the product information database with each type of perishable products associated with a respective one of the plurality of data tables.

Optionally, the method includes selecting a type of perishable product via a user interface by a user; and communicating the selection to the analysis module.

Optionally, the gas is Ethylene.

A computer implemented method of operating a cargo transport system according to an example embodiment includes measuring an environment parameter of containment air by a parameter detector; measuring a condition of a perishable product by a condition detector; sending a condition signal indicative of the condition to a control module by the condition detector; sending a parameter signal indicative of the measured environment parameter to the control module by the parameter detector; comparing the condition signal to a threshold; accessing a data table containing pre-programmed environment parameter targets based on met thresholds to determine an environment parameter target based on the met threshold association with the condition signal of the condition detector; and utilizing the threshold, the environment parameter target and the parameter signal to control an environmental control assembly thereby reaching the environment parameter target.

A computer program product for preserving perishable products stored within a containment according to another, non-limiting, embodiment includes a detector data module configured to receive and store product condition data measured by a condition detector; a product information database configured to store at least one pre-programmed product type table, wherein each product type table is associated with a respective product type of a plurality of product types; an analysis module configured to retrieve the product condition data and the product type table associated with a perishable product in the containment and determine if a threshold in the product type table is met by the condition data; and a report module configured to output a report at least when the threshold is met.

Optionally, the detector data module is configured to receive and store environment parameter data measured by a parameter detector.

Optionally, analysis module is configured to compare the met threshold to at least one environment parameter target of the product type table and output a command signal to a environmental control assembly to meet the environment parameter target.

The foregoing features and elements may be combined in various combinations without exclusivity, unless expressly indicated otherwise. These features and elements as well as the operation thereof will become more apparent in light of the following description and the accompanying drawings. However, it should be understood that the following description and drawings are intended to be exemplary in nature and non-limiting.

### BRIEF DESCRIPTION OF THE DRAWINGS

Various features will become apparent to those skilled in the art from the following detailed description of the disclosed non-limiting embodiments. The drawings that accompany the detailed description can be briefly described as follows:
FIG. 1 is a side view of a tractor trailer system as one, non-limiting, application of a cargo transport system;
FIG. 2 is a schematic of the cargo transport system;
FIG. 3 is a schematic of a product condition system of the cargo transport system;
FIG. 4 is a perspective view of a detector of the cargo transport system secured to a perishable product;
FIG. 5 is a pre-programmed product type table of the product condition system;
FIG. 6 is a second tier of the product type table; and
FIG. 7 is a flow chart of a method of operating the product condition system.

### DETAILED DESCRIPTION

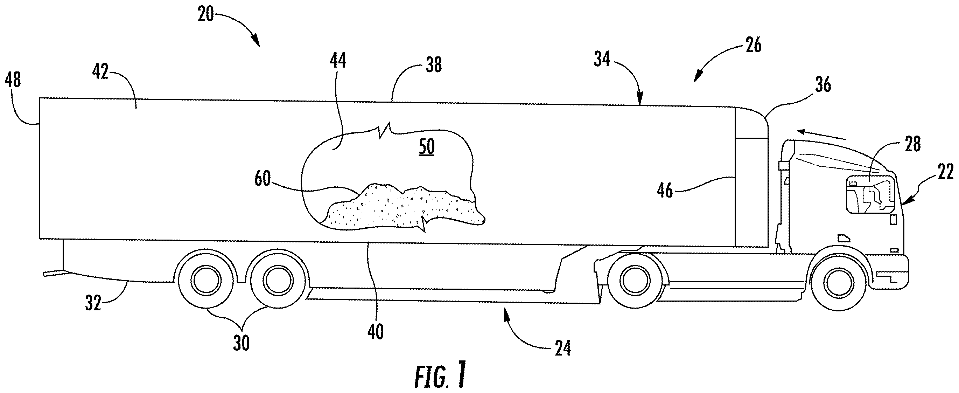

Referring to FIG. 1, one, non-limiting, application for a transport containment assembly of the present disclosure is illustrated as a tractor trailer system 20. The tractor trailer system 20 may include a tractor 22, a trailer 24 and a cargo transport system 26 utilized to control environmental parameters. The tractor 22 may include an operator's compartment or cab 28 and an engine (not shown) which is part of the powertrain or drive system of the tractor 22. The trailer 24 may include a plurality of wheels 30 rotationally engaged to a frame or platform 32 that may be detachably coupled to the tractor 22. The frame 32 is constructed to support the cargo transport system 26 for ground transport to desired destinations. The cargo transport system 26 may be an integral part of the frame 32, or, may be constructed for removal from the frame. It is contemplated and understood that the transport containment assembly 26 may be constructed for other types of transportation other than tractor trailer systems and/or may be adapted for use in multiple types of transportation (e.g., ground, sea, and/or air).

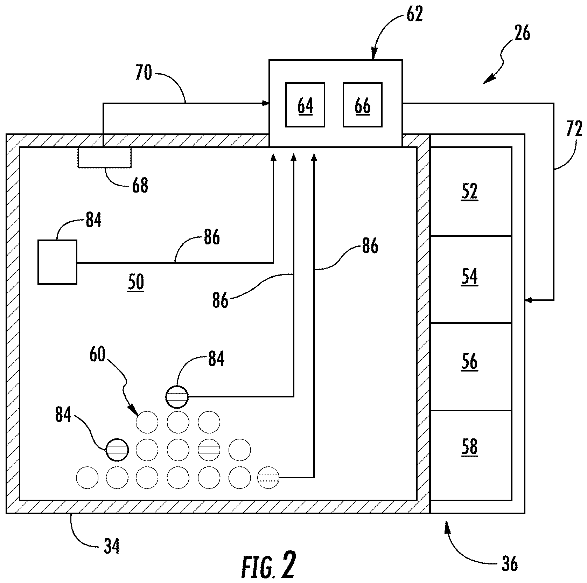

Referring to FIGS. 1 and 2, the cargo transport system 26 may include a container 34 and an environmental control assembly 36. The container 34 may include top, bottom, two sides, front and rear walls 38, 40, 42, 44, 46, 48 (also see FIG. 2) that together define the boundaries of a cargo compartment or space 50. The environmental control assembly 36 may be an integral part of the container 34 and may be located at or near the front wall 46. The environmental control assembly 36 facilitates the control of environmental parameters within the cargo compartment 50. The container 34 may further include doors (not shown) at the rear wall 48, or any other wall. It is contemplated and understood that the container 34 may be any shape and, in some applications, may not be completely enclosed (e.g., no top wall 38 and/or no side walls 42, 44, etc.).

Depending upon the environment parameter being controlled, the environmental control assembly 36 may include a refrigeration unit 52, a humidity control unit 54, an air exchange unit 56, and an environment composition control unit 58. Although illustrated separately, it is understood that any two or more of the units 52, 54, 56, 58 may generally be integrated together thereby sharing various components to achieve an end goal of controlling one or more environment parameters. For example, an environment parameter may be temperature controlled by the refrigeration unit 52. An environment parameter may be humidity controlled by the humidity control unit 54. However and depending upon outside conditions, the humidity and/or temperature may be controlled by the exchange of air accomplished via the air exchange unit 56. Another environment parameter may be a molecular composition of the air in the compartment 50. If the detected air composition is undesirable, it may be resolved via the environment composition control unit 58 that may, as one example, include a series of bottles containing one or more compressed gasses that can be injected into the compartment 50. Depending upon the cargo or product 60, the compressed gas may be an inert gas. Further examples of the compressed gas may be carbon dioxide, nitrogen, and others. Other examples of environment parameters that may be controlled include oxygen concentration, carbon dioxide concentration, ethylene concentration, ozone and 1-Methylcyclopropene (1-MCP).

A control module 62 of the environmental control assembly 36 is configured to control any one or more of the units 52, 54, 56, 58, and may include a computer-based processor 64, a computer readable and writeable storage medium 66 and at least one of any variety of environment detectors 68 as dictated by the needs and control of the various units 52, 54, 56, 58. The environment detector 68 may be configured to monitor and/or measure at least one environment parameter and output an associated signal (see arrow 70) to the control module 62. The processor 64 of the control module 62 may be configured to process the signal 70 and send an associated command signal (see arrow 72) to any one or more of the units 52, 54, 56, 58 to control and maintain any variety of environment parameters.

The environment detector 68 may be located in the containment 50 for generally measuring the environment parameter of the air in the containment which generally surrounds the product 68. The environment parameter may be dependent upon the product 60 being stored and/or transported, and may generally dictate the type of detector 68 utilized. For example, the environment detector 68 may be any one or more of a humidity sensor, a chemical sensor, a temperature sensor, oxygen sensor, carbon dioxide sensor, light sensor, ethylene sensor, ozone sensor, a shock or vibration sensor, and others. More specifically, if the environment parameter is temperature, then the environment detector 68 may be a temperature sensor. If the environment parameter is molecular composition, then the environment detector 68 may be a chemical sensor, and if the environment parameter is humidity, then the environment detector 68 may be a humidity sensor. The environment signal 70 generated by the environment detector 68 may be transmitted over a wired or wireless pathway. For example, if the control module 62 is secured to the container 34 (i.e., travels with the container), the environment detector 68 may utilize a wired pathway. If the control module 62 is remotely located (e.g., in the cab 28 or otherwise at a land-based location), the environment detector may utilize a wireless pathway.

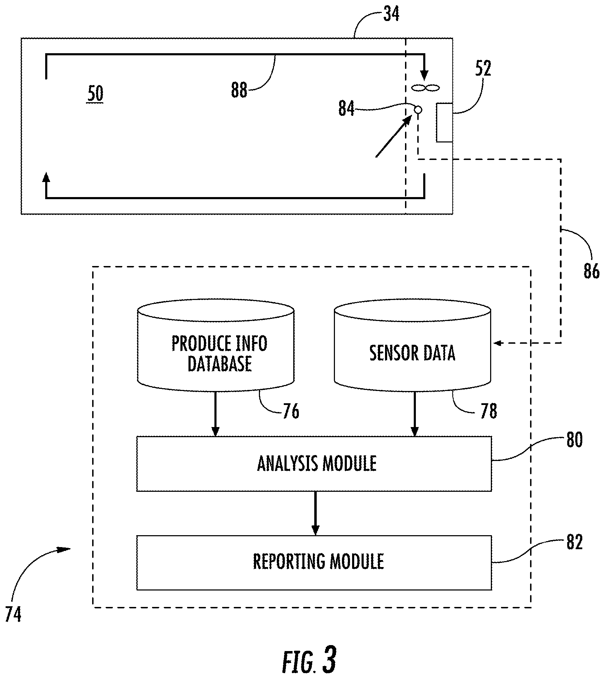

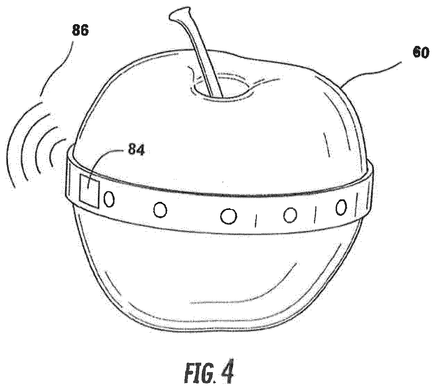

Referring to FIGS. 2 and 3, the product 60 may be a perishable product availing itself of a product condition system 74 (see FIG. 4) of the cargo transport system 26. The product condition system 74 may be adapted to monitor the perishable product 60, preserve the perishable product, and/or report out what may be a real-time condition of the perishable product. The product condition system 74 may include a pre-programmed product information database 76, a sensor data module 78, an analysis module 80, a reporting module 82, and a product condition detector 84. Except for the condition detector 84, the product condition system 74 may be substantially software-based and programmed into the control module 62. More specifically, the pre-programmed product information database 76 and the sensor data module 78 may be stored in the computer readable and writeable storage medium 66, the analysis module 80 may be part of the processor 64, and the reporting module 82 may include information outputted by the processor 64 (i.e., via processing of a condition signal 86 received by the condition detector 84), and stored in the medium 66 for later retrieval by a user. In addition to, or alternatively, the reporting module 82 may be configured to, at least in-part, send a command signal to any one of the units 52, 54, 56, 58 to alter an environment parameter to minimize or prevent degradation of the perishable product 60. It is further contemplated and understood that the product condition detector 84 may be the environment parameter detector 68, thus the detector may serve a dual purpose of providing data to the product condition system 74 indicative of a condition of the perishable product 60 and to generally monitor and control an associated environment parameter in the containment 50. It is further contemplated that the product condition system 74 may include a processor and computer readable and writeable storage medium that is separate from the control module 62, and instead, is configured to communicate with the control module 62 via, for example, the reporting module 82. The perishable product 60 may be anything capable of degrading during storage and/or transport including vegetables, fruits, meats, flowers, and other edible and non-edible products.

As illustrated in FIG. 3, the condition detector 84 may be, for example, a gas detector disposed in a supply duct 88 to the refrigeration unit 52 of the environmental control assembly 36. The product condition system 74 may be configured to monitor the levels of Ethylene and alert the refrigeration unit 52 to take corrective action when a pre-programmed Ethylene threshold value is reached that may indicate, for example, excessive ripening. Such action may be to decrease temperatures in the containment 50 as measured by the parameter detector 68. It is further contemplated and understood that, for example, the temperature in the containment may be monitored, via the parameter detector 68, and sent to the product condition system 74 for later reports, thus providing a time-based recording of both containment temperature and Ethylene levels.

In one example, the perishable product 60 may be apples. Environment parameters that may be controlled to preserve apples may include humidity, temperature, light intensity, ethylene, ethanol, and acetaldehyde levels. To preserve apples and delay the ripening process, the containment 50 may be kept at low oxygen levels of about one percent, at carbon dioxide levels of between one and five percent, at low temperatures of about zero degrees centigrade, at high humidity of about ninety to ninety-five percent, and/or at an ethylene concentration range of about one to four-hundred parts per million.

In another example, the ripeness of a banana may be controlled by controlling the temperature within the containment 50, and by controlling the airflow (i.e., air exchange) to regulate the amount of carbon dioxide and ethylene present in the containment air. The degree of ripeness may be determined and recorded by measuring the concentration of the gasses produced by the banana and found in the containment air. In this example, data from the environment detector 68 may be used and applied by the analysis module 80 supported by the processor 64 then appropriately adjusted and controlled via the environmental control assembly 36 as dictated by the command signal 72 of the control module 62.

In alternative embodiments, the condition detector 84 may be a plurality of detectors with at least one detector being proximate to a respective storage crate of a plurality of crates (not shown) stored in the containment 50. Each crate may contain a different type of perishable product. In yet another embodiment (see FIG. 4), the condition detector may be secured directly to what may be a random selection of perishable products 60. The condition detector(s) 84 may be attached directly to the perishable products to make direct, objective measurements of key condition attributes. Such measurements may include as non-limiting examples: color, firmness, and compositional changes, and/or emitted gases via respiration. Detector types may include imaging (i.e., camera), color, firmness, temperature, chemical, and others. For example, if the product composition of concern is firmness, the detector 84 may be a type of thin-film strain gauge that may further be part of a resiliently stretchable band that wraps about the perishable product 60. Another example of a condition detector 84 may include a radio frequency identification tag (RFID) with onboard gas sensing capability.

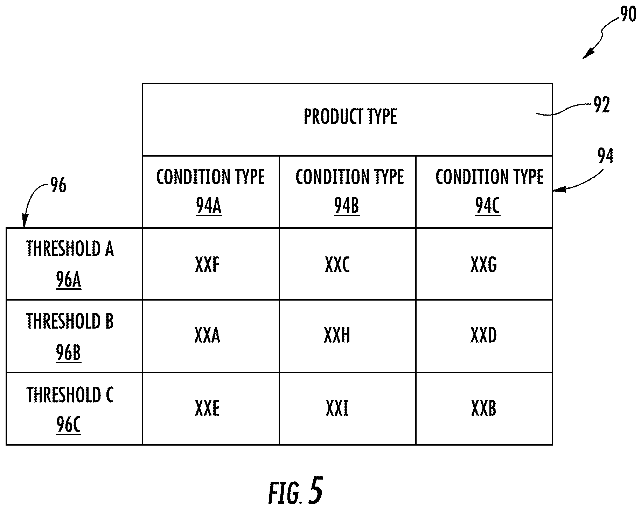

Referring to FIG. 5, the product information database 76 may include a plurality of tables 90 with each table being pre-programmed and specific to a type 92 of the perishable product 60. For the product type 92, the table 90 may include at least one product condition type 94 (i.e. three illustrated as 94A, 94B, and 94C). Non-limiting examples of condition types 94 may include color, firmness, gas concentration, and others. For each condition type 94, the product information database 76 may store at least one threshold value or range 96 (i.e. three illustrated as 96A, 96B, and 96C), pre-established to enact a specific action such as for example, transmit an alert to a user via the reporting module 82, and/or take a corrective action that may include initiation of the environmental control assembly 36. It is further contemplated and understood that a particular table 90 may be pre-selected by a user via a user interface (not shown) that communicates with the product condition system 74 once the user knows the type of product being stored and/or transported. Furthermore, the system 74 may be configured to know which type(s) of condition detectors 84 are available or pre-configured with a particular container 34, and thereby, automatically selects the correlating condition type 94 associated with the type of detector.

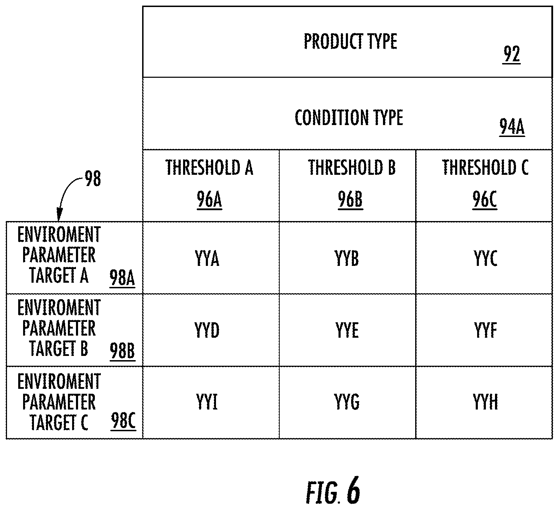

Referring to FIGS. 5 and 6, each table of a particular product type 92 and a particular condition type 94 may include at least one environment parameter target 98 (i.e. three illustrated as targets 98A, 98B, 98C). Environmental parameter targets 98 may be a desired value and/or a desired range of values that are preferred in order to preserve a perishable product and/or inhibit degradation (or further degradation) of a perishable product 60. What the environment parameter target 98 is may be dependent on the particular thresholds 96A, 96B, 96C that may be representative of the condition of the particular product type 92 of the perishable product 60. Examples of environment parameter targets 98 may include containment temperature, humidity, gas concentrations, rate of air exchanges, and others.

Referring to FIGS. 2-3 and 5-6, the storage medium 66 may: store algorithms executed by the analysis module 80 of the processor 64; may store detector 68, 84 data accumulated during storage and/or transit of the particular product 60; and may further store the data tables 92 specific to the type of product 60. For example, if the perishable product 60 is bananas, the 'banana' data table 90 may include desired environment parameter ranges or targets 98 needed to preserve and/or prevent the bananas from ripening or ripening too fast. Such data may include temperature, humidity, and the presence of certain gases (e.g., carbon dioxide and ethylene) which are produced during the ripening process.

Applying the relevant data tables 90, the analysis module 80 once receiving the parameter and/or condition signals 70, 86 may execute associated algorithms to first determine relevant and desired environment parameter target(s) 98, and may then generate appropriate command signals 72 that are sent to the environmental control assembly 36. The environmental control assembly 36 may then initiate the appropriate unit(s) 52, 54, 56, 58 to adjust the measured environment parameter of the containment air. It is contemplated and understood that the measured environment parameter and the measured product condition may be functions of the algorithm.

In another example, the measured product condition may be a function of the algorithm and the measured environment parameter is used to directly control the environmental control assembly 36. That is, combinations of 'targeted' environment parameters may be based on current conditions and needs of the perishable product 60 and would affect the product in various manners including slowing or accelerating ripening, inhibiting post-harvest plant pathogen growth, inhibiting water loss, inhibiting or promoting color change, and/or adjusting to changes in chilling sensitivity. It is understood that the term 'targeted' environment parameter is that parameter calculated by the control module 66 based on real-time conditions of the product 60. Via the command signal 72, it is the goal of the environmental control assembly 36 to adjust toward or obtain the parameter target or value 98. It is further contemplated and understood that this process may conserve energy since the environmental control assembly 36 may operate in real-time and consume energy only when needed (i.e., current needs).

Although not specifically illustrated in the data tables of FIGS. 5 and 6, it is further contemplated and understood that through executable algorithms, any one or more of the thresholds 96 may be dependent upon two or more condition types 94. Similarly, the environment parameter target 98 may be dependent upon two or more thresholds 96 of multiple condition types 94 and a particular product type 92. Although such calculations may require an increase in the number and diversity of detectors, by establishing a threshold 96 via a function of multiple condition types 94; and/or, establishing an environment parameter target 98 through a function of multiple thresholds 96 of different condition types 94, the reliability and accuracy of the product condition system 74 may be optimized.

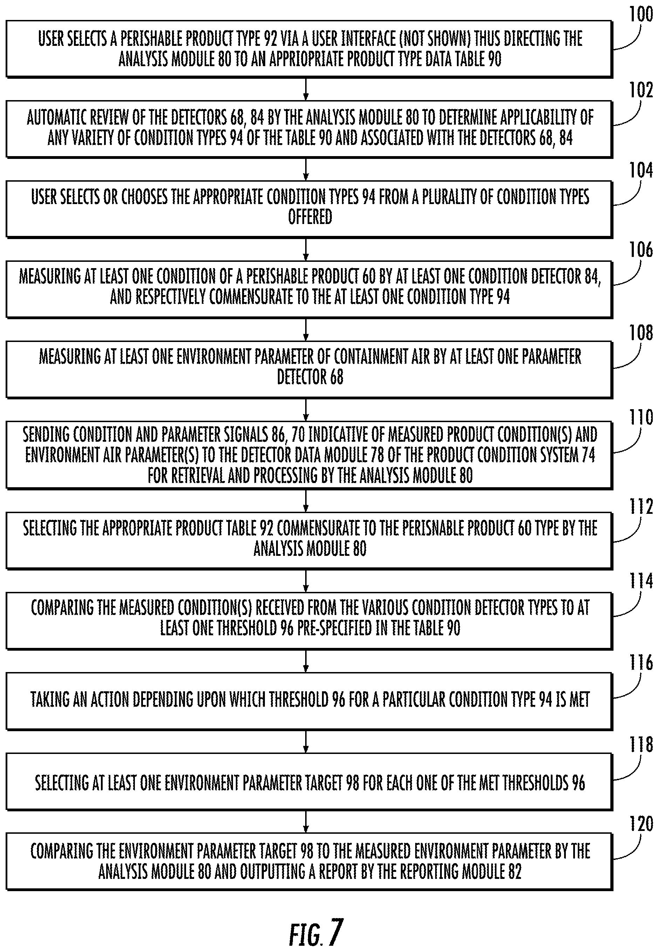

Referring to FIG. 7, a method of operating the cargo transport system 26 includes a first block 100 of a user selecting a perishable product type 92 via a user interface (not shown) thus directing the analysis module 80 to an appropriate product type data table 90. Block 102 entails an automatic review of the detectors 68, 84 by the analysis module 80 to determine applicability of any variety of condition types 94 of the table 90 and associated with the detectors 68, 84. In addition to, or alternatively, to block 102, block 104 includes the user selecting or choosing the appropriate condition types 94 from a plurality of condition types offered.

Block 106 includes measuring at least one condition of a perishable product 60 by at least one condition detector 84, and respectively commensurate to the at least one condition type 94. A block 108 entails measuring at least one environment parameter of containment air by at least one parameter detector 68. Block 110 entails sending condition and parameter signals 86, 70 indicative of measured product condition(s) and environment air parameter(s) to the detector data module 78 of the product condition system 74 for retrieval and processing by the analysis module 80.

Block 112 entails selecting the appropriate product table 92 commensurate to the perishable product 60 type by the analysis module 80. Block 114 entails comparing the measured condition(s) received from the various condition detector types to at least one threshold 96 pre-specified in the table 90. Block 116 entails taking an action depending upon which threshold 96 for a particular condition type 94 is met.

For a particular product type 92 and a particular condition type 94, block 118 entails selecting at least one environment parameter target 98 for each one of the met thresholds 96. Block 120 entails comparing the environment parameter target 98 to the measured environment parameter by the analysis module 80 and outputting a report by the reporting module 82, wherein the report may be a command signal 72 to the appropriate/associated unit 52, 54, 56, 58 of the environmental control assembly 36 to reach the associated/respective target.

The present disclosure may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Smalltalk, C++ or the like, and conventional procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Benefits and advantages of the present disclosure include an objective assessment of actual perishable product condition over a time span during, for example, transportation. Other advantages include a real-time feedback to the transport refrigeration unit (TRU), an intelligent manipulation of environmental parameters to optimize the condition of the perishable products upon arrival, minimize wear on the TRU, and optimizing energy efficiency. Yet further, because current conditions of the perishable product is known during transit, real-time technical, operational and commercial decision making can be achieved.

While the present disclosure is described with reference to illustrated embodiments, it will be understood by those skilled in the art that various changes may be made and equivalents may be substituted without departing from the scope of the present invention, as defined by the claims. In addition, various modifications may be applied to adapt the teachings of the present disclosure to particular situations, applications, and/or materials. The present invention is thus not limited to the particular examples disclosed herein, but includes all embodiments falling within the scope of the appended claims.

## Claims

1. A computer implemented method of operating a product condition system (74) comprising:
measuring first and second conditions of a perishable product (60) by respective first and second detectors (84), wherein the first condition of the perishable product is a gas emitted by the perishable product and the first detector is a gas detector, and a first condition signal associated with the measured first condition of the perishable product is sent from the first detector and to a detector data module (78), and wherein the second condition of the perishable product is a different condition type to that of the first condition of the perishable product;
measuring a first environment parameter of a containment (50) where the perishable product is located, wherein the first environment parameter is measured by an environment detector (68);
comparing the first and second conditions of the perishable product to respective first and second pre-programmed thresholds (96) respectively;
determining a single environment parameter target (98) when at least one of the first and second pre-programmed (96) thresholds are met from a pre-programmed data table (90) as a function of both of the measured first and second measured conditions of the perishable product, wherein the single environment parameter target is established to reduce degradation of the perishable product, and wherein the determining comprises accessing the pre-programmed data table indicative of a perishable product type (92) by an analysis module (80) and from a product information database (76), wherein the pre-programmed data table (90) includes a plurality of condition types (94), a plurality of thresholds (96) associated with each condition type of the plurality of condition types; and
sending a command signal (72) derived from the single environmental parameter target to an environmental control assembly (36) to meet the first environment parameter target.

2. The computer implemented method set forth in claim 1, wherein the analysis module (80) and the reporting module (82) are software-based.

3. The computer implemented method set forth in claim 2, wherein the detector data module (78) and the product information database (76) are contained within a computer readable and writeable storage medium.

4. The computer implemented method set forth in claim 3, wherein the analysis module (80) is supported by a microprocessor.

5. The computer implemented method set forth in claim 1 further comprising:
measuring the second condition of the perishable product (60) by the second detector (84); and
comparing the second condition of the perishable product to a second pre-programmed threshold (96).

6. The computer implemented method set forth in claim 5 further comprising:
determining a second environment parameter target (98) when the second pre-programmed threshold (96) is met.

7. The computer implemented method set forth in claim 1, wherein the perishable product (60) is one type of a plurality of types (92) of perishable products, and the data table (90) is one of a plurality of data tables stored in the product information database (76) with each type of perishable products associated with a respective one of the plurality of data tables.

8. The computer implemented method set forth in claim 7 further comprising:
selecting a type of perishable product (60) via a user interface by a user; and
communicating the selection to the analysis module (80).

9. The computer implemented method set forth in claim 1, wherein the gas is Ethylene.

10. A computer implemented method according to any preceding claim for operating a cargo transport system.

## Patentansprüche

1. Computerimplementiertes Verfahren zum Betreiben eines Produktzustandssystems (74), umfassend:
Messen erster und zweiter Zustände eines verderblichen Produkts (60) durch jeweilige erste und zweite Detektoren (84), wobei der erste Zustand des verderblichen Produkts ein Gas ist, das von dem verderblichen Produkt emittiert wird, und der erste Detektor ein Gasdetektor ist, und ein erstes Zustandssignal, das dem gemessenen ersten Zustand des verderblichen Produkts zugeordnet ist, von dem ersten Detektor und zu einem Detektordatenmodul (78) gesendet wird, und wobei der zweite Zustand des verderblichen Produkts eine unterschiedliche Art von Zustand zu dem des ersten Zustands des verderblichen Produkts ist;
Messen eines ersten Umgebungsparameters eines Behälters (50), in dem sich das verderbliche Produkt befindet, wobei der erste Umgebungsparameter von einem Umgebungsdetektor (68) gemessen wird;
Vergleich des ersten und zweiten Zustands des verderblichen Produkts mit jeweils ersten und zweiten vorprogrammierten Schwellenwerten (96);
Bestimmen eines einzelnen Umgebungsparameterziels (98), wenn mindestens einer der ersten und zweiten vorprogrammierten (96) Schwellenwerte aus einer vorprogrammierten Datentabelle (90) als Funktion von beiden der gemessenen ersten und zweiten gemessenen Zustände des verderblichen Produkts erfüllt wird, wobei das einzelne Umgebungsparameterziel eingerichtet wird, um die Verschlechterung des verderblichen Produkts zu verringern, und wobei das Bestimmen Zugreifen auf die vorprogrammierte Datentabelle, die einen verderblichen Produkttyp (92) angibt, durch ein Analysemodul (80) und von einer Produktinformationsdatenbank (76) umfasst, wobei die vorprogrammierte Datentabelle (90) eine Vielzahl von Arten von Zustand A(94), eine Vielzahl von Schwellenwerten (96), die jeder Art von Zustand der Vielzahl von Arten von Zustand zugeordnet sind, beinhaltet; und
Senden eines von dem einzelnen Umgebungsparameterziel abgeleiteten Befehlssignals (72) an eine Umgebungssteuerungsbaugruppe (36), um das erste Umgebungsparameterziel zu erfüllen.

2. Computerimplementiertes Verfahren nach Anspruch 1, wobei das Analysemodul (80) und das Berichtsmodul (82) softwarebasiert sind.

3. Computerimplementiertes Verfahren nach Anspruch 2, wobei das Detektordatenmodul (78) und die Produkt-Informationsdatenbank (76) in einem computerlesbaren und beschreibbaren Speichermedium enthalten sind.

4. Computerimplementiertes Verfahren nach Anspruch 3, wobei das Analysemodul (80) durch einen Mikroprozessor unterstützt wird.

5. Computerimplementiertes Verfahren nach Anspruch 1, ferner umfassend:
Messen des zweiten Zustands des verderblichen Produkts (60) durch den zweiten Detektor (84); und
Vergleichen des zweiten Zustands des verderblichen Produkts mit einem zweiten vorprogrammierten Schwellenwert (96).

6. Computerimplementiertes Verfahren nach Anspruch 5, ferner umfassend:
Bestimmen eines zweiten Umgebungsparameterziels (98), wenn der zweite vorprogrammierte Schwellenwert (96) erfüllt ist.

7. Computerimplementiertes Verfahren nach Anspruch 1, wobei das verderbliche Produkt (60) eine Art aus einer Vielzahl von Arten (92) verderblicher Produkte ist und die Datentabelle (90) eine aus einer Vielzahl von Datentabellen ist, die in der Produktinformationsdatenbank (76) gespeichert ist, wobei jede Art von verderblichen Produkten einer entsprechenden aus der Vielzahl von Datentabellen zugeordnet ist.

8. Computerimplementiertes Verfahren nach Anspruch 7, ferner umfassend:
Auswählen einer Art von verderblichem Produkt (60) über eine Benutzerschnittstelle durch einen Benutzer; und
Kommunizieren die Auswahl an das Analysemodul (80).

9. Computerimplementiertes Verfahren nach Anspruch 1, wobei das Gas Ethylen ist.

10. Computerimplementiertes Verfahren nach einem der vorhergehenden Ansprüche zum Betreiben eines Frachttransportsystems.

## Revendications

1. Procédé mis en œuvre par ordinateur de fonctionnement d'un système d'état de produit (74) comprenant :
la mesure de premier et second états d'un produit périssable (60) par des premier et second détecteurs (84) respectifs, dans lequel le premier état du produit périssable est un gaz émis par le produit périssable et le premier détecteur est un détecteur de gaz, et un premier signal d'état associé au premier état mesuré du produit périssable est envoyé depuis le premier détecteur et à un module de données de détecteur (78), et dans lequel le second état du produit périssable est un type d'état différent de celui du premier état du produit périssable ;
la mesure d'un premier paramètre environnemental d'une enceinte de confinement (50) où se trouve le produit périssable, dans lequel le premier paramètre environnemental est mesuré par un détecteur environnemental (68) ;
la comparaison des premier et second états du produit périssable à des premier et second seuils préprogrammés (96) respectifs ;
la détermination d'une cible de paramètre d'environnement unique (98) lorsqu'au moins l'un des premier et second seuils préprogrammés (96) est atteint à partir d'une table de données préprogrammée (90) en fonction à la fois des premier et second états mesurés du produit périssable, dans lequel la cible de paramètre d'environnement unique est établie pour réduire la dégradation du produit périssable, et dans lequel la détermination comprend l'accès à la table de données préprogrammée indiquant un type de produit périssable (92) par un module d'analyse (80) et à partir d'une base de données d'informations de produit (76), dans lequel la table de données préprogrammée (90) comporte une pluralité de types d'état (94), une pluralité de seuils (96) associés à chaque type d'état de la pluralité de types d'état ; et
l'envoi d'un signal de commande (72) dérivé de la cible de paramètre environnemental unique à un ensemble de contrôle environnemental (36) pour atteindre la première cible de paramètre environnemental.

2. Procédé mis en œuvre par ordinateur selon la revendication 1, dans lequel le module d'analyse (80) et le module de rapport (82) sont basés sur un logiciel.

3. Procédé mis en œuvre par ordinateur selon la revendication 2, dans lequel le module de données de détecteur (78) et la base de données d'informations de produit (76) sont contenus dans un support de stockage lisible et inscriptible par ordinateur.

4. Procédé mis en œuvre par ordinateur selon la revendication 3, dans lequel le module d'analyse (80) est supporté par un microprocesseur.

5. Procédé mis en œuvre par ordinateur selon la revendication 1, comprenant également :
la mesure du second état du produit périssable (60) par le second détecteur (84) ; et
la comparaison du second état du produit périssable à un second seuil préprogrammé (96) .

6. Procédé mis en œuvre par ordinateur selon la revendication 5, comprenant également :
la détermination d'une seconde cible de paramètre d'environnement (98) lorsque le second seuil préprogrammé (96) est atteint.

7. Procédé mis en œuvre par ordinateur selon la revendication 1, dans lequel le produit périssable (60) est un type d'une pluralité de types (92) de produits périssables, et la table de données (90) est l'une d'une pluralité de tables de données stockées dans la base de données d'informations de produit (76), chaque type de produit périssable étant associé à une table respective de la pluralité de tables de données.

8. Procédé mis en œuvre par ordinateur selon la revendication 7, comprenant également :
la sélection d'un type de produit périssable (60) par l'intermédiaire d'une interface utilisateur par un utilisateur ; et
la communication de la sélection au module d'analyse (80).

9. Procédé mis en œuvre par ordinateur selon la revendication 1, dans lequel le gaz est l'éthylène.

10. Procédé mis en œuvre par ordinateur selon une quelconque revendication précédente de fonctionnement d'un système de transport de marchandises.
